# FASCICULE DE BREVET EUROPEEN

(11) **EP 0 701 431 B1**
(45) Date de publication et mention de la délivrance du brevet: **17.09.1997**
(21) Numéro de dépôt: 94917703.4
(22) Date de dépôt: 01.06.1994
(51) Int. Cl.: A61K 7/50, A61K 7/06, A61K 7/48

(54) **COMPOSITIONS COSMETIQUES CONTENANT AU MOINS UN TENSIO-ACTIF ANIONIQUE DU TYPE ALKYLGALACTOSIDE URONATE ET AU MOINS UN POLYMERE CATIONIQUE, ET LEURS UTILISATIONS POUR LE TRAITEMENT DES MATIERES KERATINIQUES**
ANIONISCHE ALKYLGALAKTOSIDURONATE-TENSIDE UND KATIONISCHE POLYMERE ENTHALTENDE KOSMETISCHE ZUSAMMENSETZUNGEN UND IHRE VERWENDUNG ZUR BEHANDLUNG VON KERATINÖSEN SUBSTANZEN
COSMETIC COMPOUNDS CONTAINING AT LEAST ONE ANIONIC ALKYLGALACTOSIDE URONATE TYPE SURFACE-ACTIVE AGENT AND AT LEAST ONE CATIONIC POLYMER AND THEIR USE IN THE TREATMENT OF KERATINOUS MATERIALS

(30) Priorité: 01.06.1993 FR 9306531
(43) Date de publication de la demande: 20.03.1996
(73) Titulaire: L'OREAL, 75008 Paris (FR)
(72) Inventeur: CAUWET, Danièle, F-75011 Paris (FR); DUBIEF, Claude, F-78150 Le Chesnay (FR)
(74) Mandataire: Casalonga, Axel
(86) Numéro de dépôt international: FR9400642
(87) Numéro de publication internationale: WO9427575

(56) Documents cités:
- EP-A- 0 275 153
- EP-A- 0 337 354
- EP-A- 0 550 276
- WO-A-92/10162
- WO-A-93/02092
- FR-A- 2 197 567

## Description

L'invention concerne des compositions cosmétiques contenant au moins un tensio-actif anionique du type alkylgalactoside uronate et au moins un polymère cationique et leur utilisation pour le traitement et le lavage des matières kératiniques.

Les compositions de lavage des cheveux ou de la peau sont généralement formulées à partir d'agents tensio-actifs anioniques ou non-ioniques ou leurs mélanges en présence éventuellement d'agents tensio-actifs amphotères.

Les cheveux agressés par les agents atmosphériques tels que la lumière ou les traitements chimiques et lavés avec les compositions de lavage classiques sont difficilement démêlables et cet inconvénient se trouve encore accentué dans le cas de cheveux mouillés.

On a proposé d'ajouter à de telles bases lavantes des polymères cationiques pour améliorer les propriétés de démêlage, mais le volume et la quantité des mousses notamment leur douceur et leur compacité, se sont pas satisfaisants.

Les tensio-actifs anioniques du type alkylgalactoside uronate ont déjà été préconisés dans des compositions lavantes pour les cheveux. Ils ont été décrits dans la demande de brevet EP-A-0 532 370.

Les compositions de lavage des cheveux utilisant ces tensio-actifs anioniques ou non-ioniques seuls ne conduisent pas à de bonnes propriétés cosmétiques en particulier le démêlage des cheveux mouillés est difficile.

La demanderesse vient de découvrir de manière surprenante, que l'association, dans des compositions lavantes et/ou traitantes pour matières kératiniques, d'un tensio-actif anionique du type alkylgalactoside uronate et d'un polymère cationique conférait à ces compositions des propriétés de démêlage considérablement améliorées, en particulier des cheveux humides.

Par ailleurs, l'association conforme à la présente invention permet d'obtenir une mousse abondante compacte et très douce.

En outre, la demanderesse a constaté que les compositions cosmétiques contenant une telle association confèrent aux matières kératiniques de bonnes propriétés cosmétiques telles que la douceur, un toucher agréable.

La présente invention a donc pour objet des compositions cosmétiques contenant au moins un agent tensio-actif anionique du type alkylgalactoside uronate et au moins un polymère cationique à l'exclusion des éthers de celluloses cationiques.

Un autre objet de l'invention est constitué par l'utilisation de ces compositions pour le traitement et/ou le lavage des matières kératiniques telles que les cheveux ou la peau.

Un autre objet concerne des procédés de traitements cosmétiques des cheveux ou de la peau au moyen des compositions de l'invention; les procédés de lavage et de traitement des cheveux étant préférés.

Les compositions cosmétiques selon l'invention contiennent dans un milieu aqueux cosmétiquement acceptable, au moins un alkylgalactoside uronate et au moins un polymère cationique à l'exclusion des celluloses cationiques telles que les éthers de celluloses cationiques, les celluloses quaternisées.

Les alkylgalactoside uronates, utilisables conformément à l'invention, répondent à la formule (I) suivante : 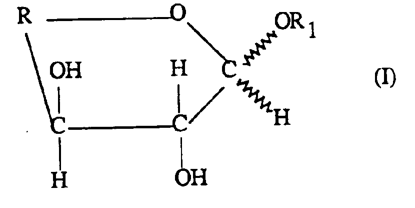 dans laquelle :
R₁ désigne un radical alkyle linéaire ou ramifié de 8 à 22 atomes de carbone.
R désigne un groupe
   (i) ;CH-CH(OH)-C02R2 ou
   (ii)
      - CH(OH)-ÇH-C0₂R₂

   dont le carbone portant le groupe hydroxyle est relié à l'atome d'oxygène endocyclique; R₂ étant l'hydrogène, un métal alcalin, un métal alcalino-terreux ou un groupe ammonium quaternaire substitué ou non par des radicaux alkyle, hydroxyalkyle ou dérivé d'un amino acide, à la condition que, lorsque le polymère cationique de la composition cosmétique n'est pas une protéine quaternisée, R₂ soit différent de H et Na.

Les tensio-actifs anioniques du type alkylgalactoside uronate de formule (I) sont connus et peuvent être préparés selon les procédés décrits dans la demande de brevet EP-A-0 532 370.

Le métal alcalin est notamment le sodium, le potassium et le métal alcalino-terreux est de préférence le magnésium. Comme sels d'ammonium quaternaires, on peut citer les sels d'ammoniaque, de triéthanolamine, de monoétha- nolamine, de 2-amino 2-méthyl 1,3 propanediol, de 2-amino 2-méthyl 1-propanol; l'amino acide est notamment l'histidine, l'arginine ou la lysine.

On utilise de préférence les composés de formule (I) pour lesquels le radical R₁ désigne un alkyle en C₈-C₁₄ et plus particulièrement le radical décyle.

On utilise notamment les composés suivants :
Décyl a-D-galactopyranoside uronate de sodium : 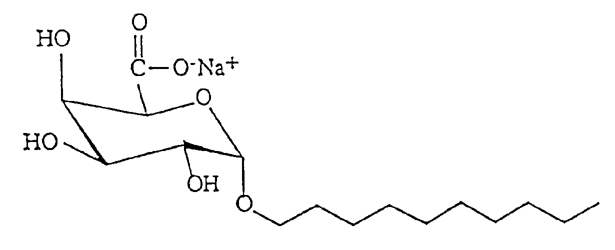
Décyl β-D-galactopyranoside uronate de sodium : 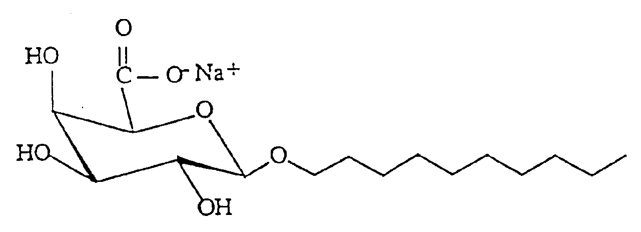
Décyl a-D-galactofuranoside uronate de sodium : 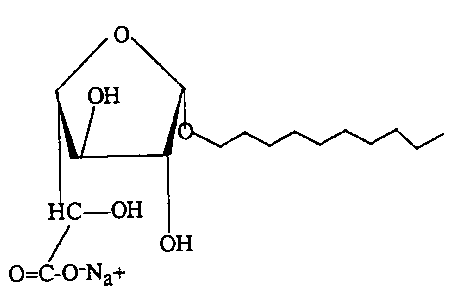
Décyl β-D-galactofuranoside uronate de sodium :

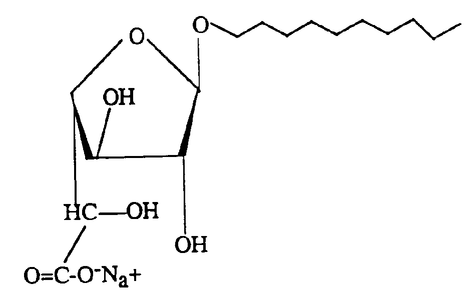

Les polymères cationiques utilisables conformément à l'invention sont choisis parmi les polymères comportant des groupements amines primaire, secondaire, tertiaire et/ou quaternaire faisant partie de la chaîne polymère ou directement reliés à celle-ci, ayant un poids moléculaire compris entre 500 et environ 5 000 000 et de préférence entre 1 000 et 3 000 000, à l'exclusion des celluloses cationiques telles que les éthers de celluloses cationiques, les celluloses quaternisées.

Parmi ces polymères on peut citer plus particulièrement les protéines quaternisées et les polymères du type polyamine, polyaminoamide, ou du type polyammonium quaternaire dont le groupe ammonium fait partie d'un cycle ou de la chaîne polymérique ou est rattaché à la chaîne polymérique par un radical hydrocarboné.

Les protéines quaternisées sont en particulier des polypeptides modifiés chimiquement et portant en bout de chaîne ou greffés sur celle-ci, des groupements ammonium quaternaires. Parmi ces protéines on peut citer notamment
- les hydrolysats de collagène portant des groupements triéthylammonium tels que les produits vendus sous la dénomination "QUAT-PRO E" par la société MAYBROOK et dénommés dans le dictionnaire CTFA "Triethonium Hydrolyzed Collagen Ethosulfate";
- les hydrolysats de collagène portant des groupements chlorure de triméthylammonium ou de diméthylstéarylam- monium tels que les produits vendus sous la dénomination de "QUAT-PRO S" par la société MAYBROOK et dénommés dans le dictionnaire CTFA "Steartrimonium Hydrolyzed Collagen";
- les hydrolysats de protéines animales portant des groupements diméthylbenzylammonium tels que les produits vendus sous la dénomination "CROTEIN BTA" par la société CRODA et dénommés dans le dictionnaire CTFA "Benzyltrimonium Hydrolyzed Animal Protein";
- les hydrolysats de protéines portant sur la chaîne polypeptidique des groupements ammonium quaternaires comportant au moins un radical alkyle ayant de 1 à 18 atomes de carbone. Parmi ces hydrolysats de protéines, on peut citer entre autres :

le CROQUAT L dont la chaîne polypeptidique a un poids moléculaire moyen d'environ 2500 et dont le groupement ammonium quaternaire comporte un groupement alkyle en C₁₂;
le CROQUAT M dont la chaîne polypeptidique a un poids moléculaire moyen d'environ 2500 et dont le groupement ammonium quaternaire comporte un groupement alkyle en C₁₀-C₁₈;
• le CROQUAT S dont la chaîne polypeptidique a un poids moléculaire moyen d'environ 2700 et dont le groupement ammonium quaternaire comporte un groupement alkyle en C↑ε;
la CROTEIN Q dont la chaîne polypeptidique a un poids moléculaire moyen de l'ordre de 12 000 et dont le groupement ammonium quaternaire comporte au moins un groupe alkyle ayant de 1 à 18 atomes de carbone.
Les protéines végétales quaternisées telles que la protéine quaternisée végétale de soja vendue sous la dénomination CROQUAT SOJA.

Ces différents produits sont vendus par la société CRODA.

D'autres protéines quaternisées sont celles répondant à la formule: 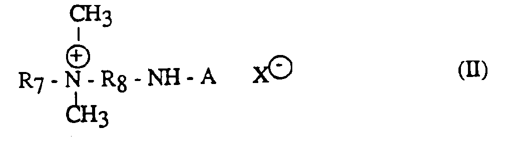 dans laquelle A désigne un reste de protéine dérivé d'hydrolysats de protéine de collagène, R₇ désigne un groupement lipophile comportant jusqu'à 30 atomes de carbone, R₈ représente un groupement alkylène ayant 1 à 6 atomes de carbone, représente un anion dérivé d'un acide organique ou minéral; ce XO otéines ont un poids moléculaire compris entre 1500 et 10 000 de préférence 2000 et 5000. Les produits préférés sont ceux vendus sous la dénomination "LEXEIN QX 3000" par la Société INOLEX appelée dans le dictionnaire CTFA "Cocotrimonium Collagen Hydrolysate".

Les polymères du type polyamine, polyaminoamide ou polyammonium quaternaire, utilisables conformément à la présente invention sont décrits en particulier dans les brevets français de la demanderesse n° 2 505 348 ou 2 542 997.

Parmi ces polymères on peut citer :
(1) les copolymères vinyl-pyrrolidonelacrylate ou méthacrylate de dialkylaminoalkyle quaternisés ou non tels que les produits vendus sous la dénomination "GAFQUAT" par la Société GAF CORPORATION comme par exemple "GAFQUAT 734 ou 755" ou bien les produits dénommés "COPOLYMER 845, 958 et 937". Ces polymères sont décrits en détail dans le brevet français 2 077 143 et 2 393 573.
(2) Les polysaccharides cationiques non cellulosiques décrits plus particulièrement dans les brevets américains 3 589 578 et 4 031 307 et plus particulièrement le produit commercialisé sous la dénomination "JAGUAR C. 13 S" vendu par la société MEYHALL.
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques ainsi que les produits d'oxydation et/ou de quaternisation de ces polymères. De tels polymères sont décrits dans les brevets français 2 162 025 et 2 280 361.
(4) Les polyaminoamides solubles dans l'eau préparés en particulier par polycondensation d'un composé acide avec une polyamine. Ces polyaminoamides peuvent être réticulés par une épihalohydrine, un diépoxyde, un dianhydride, un anhydride non saturé, un dérivé bis-insaturé, une bis-halohydrine, un bis-azétidinium, une bis-haloacyldiamine, un bis-halogènure d'alcoyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé; l'agent réticulant étant utilisé dans des proportions allant de 0,025 à 0,35 mole par groupement amine du polyaminoamide.

Ces polyaminopolyamides peuvent être alcoylés ou s'ils comportent une ou plusieurs fonctions amines tertiaires, quaternisés. De tels polymères sont décrits en particulier dans les brevets français 2 252 840 et 2 368 508. (5) Les dérivés de polyaminoamides résultant de la condensation de polyalcoylène polyamines avec des acides polycarboxyliques suivie d'une alcoylation par des agents bifonctionnels. On peut citer par exemple les polymères acide adipiquedialcoylaminohydroxyalcoyldialcoylène triamine dans lesquels le radical alcoyle comporte de 1 à 4 atomes de carbone et désigne de préférence méthyle, éthyle, propyle. De tels polymères sont décrits dans le brevet français 1 583 363.

Parmi ces dérivés, on peut citer plus particulièrement les polymères acide adipique/diméthylamino hydroxy- propyl/diéthylènetriamine vendus sous les dénomination "CARTARETINE F, F₄ ou F₈" par la Société SANDOZ. (6) Les polymères obtenus par réaction d'une polyalkylène polyamine comportant deux groupements amine primaire et au moins un groupement amine secondaire avec un acide dicarboxylique choisi parmi l'acide diglycolique, et des acides dicarboxyliques aliphatiques saturés ayant de 3 à 8 atomes de carbone. Le rapport molaire entre la polyalkylène polyamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont décrits en particulier dans les brevets américains 3 227 615 et 2 961 347.

Des polymères de ce type sont en particulier commercialisés sous la dénomination "HERCOSETT 57" par la Société HERCULES INCORPORATED ou bien sous la dénomination de "PD 170" ou "DELSETTE 101" par la Société HERCULES dans le cas du copolymère d'acide adipique/époxypropyl/diéthylène-triamine. (7) Les cyclopolymères ayant un poids moléculaire de 20 000 à 3 000 000 tels que des homopolymères comportant comme constituant principal de la chaîne des motifs répondant aux formules (III) ou (III'). 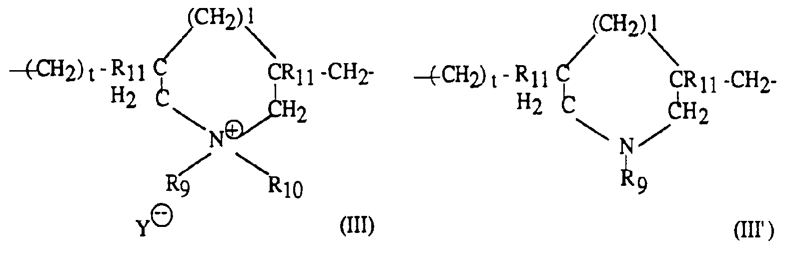 1 et t sont égaux à 0 ou 1, et la somme 1 + t = 1 , R₁₁désigne hydrogène ou méthyle, Rg et R₁₀ désignent indépendamment l'un de l'autre, un groupement alcoyle ayant de 1 à 22 atomes de carbone, un groupement hydroxyalcoyle dans lequel le groupement alcoyle a de préférence 1 à 5 atomes de carbone, un groupement amidoalcoyle inférieur, ou R₁₉ et R₁₀ peuvent former conjointement avec l'atome d'azote auquel ils sont rattachés des groupements hétérocycliques tels que pipéridinyle ou morpholinyle, ainsi que les copolymères comportant des unités de formule (III) ou (III') et des unités dérivées d'acrylamide ou de diacétone acrylamide, Y est un anion tel que bromure, chlorure, acétate, borate, citrate, tartrate, bisulfate, bisulfite, sulfate, phosphate. Parmi les copolymères définis ci-dessus on peut citer plus particulièrement l'homopolymère de chlorure de diméthyl diallyl ammonium vendu sous la dénomination "MERQUAT 100" ayant un poids moléculaire inférieur à 100 000 et le copolymère de chlorure de diméthyl diallyl ammonium et d'acrylamide ayant un poids moléculaire supérieur à 500 000 et vendu sous la dénomination de "MERQUAT 550" et "MERQUAT S" par la Société MERCK.

Ces polymères sont décrits plus particulièrement dans le brevet français 2 080 759 et son certificat d'addition n° 2 190 406.
(8) Les homopolymères ou copolymères dérivés d'esters ou d'amides acrylique ou méthacrylique et comportant les motifs : 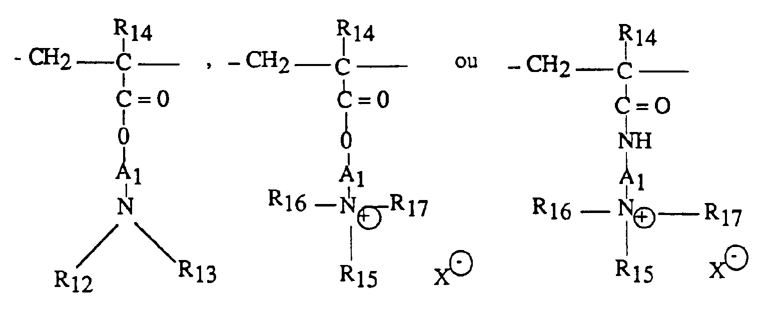 dans lesquels
R₁₄ désigne un atome d'hydrogène ou un radical méthyle A₁ est un groupe alkylène linéaire ou ramifié de 1 à 6 atomes de carbone ou un groupe hydroxyalkylène de 1 à 4 atomes de carbone,
R_{15,} R₁₆ et R₁₇ identiques ou différents représentent un groupe alkyle ayant de 1 à 18 atomes de carbone ou un radical benzyle,
R₁₂ et R₁₃ représentent un atome d'hydrogène ou un groupe alkyle ayant de 1 à 6 atomes de carbone, désigne un anion méthosulfate ou un halogénure tel que chlorure X⊖ bromure.

Le ou les comonomères utilisabes appartiennent à la famille des : acrylamide, méthacrylamide, diacétone acrylamide, acrylamide et méthacrylamide substitués à l'azote par des alkyles inférieurs, des esters d'acides acrylique ou méthacrylique, la vinylpyrrolidone, des esters vinyliques, le vinylcaprolactame.

Parmi ces composés, on peut citer le copolymère d'acrylamide et de diméthylaminoéthyl méthacrylate quater- nisé au sulfate de diméthyle et vendu sous la dénomination "HERCOFLOC" par la Société HERCULES, le copolymère d'acrylamide et de chlorure de méthacryloyloxyéthyltriméthylammonium décrit dans la demande de brevet EP-A-80976 et vendu sous la dénomination "BINA QAT P100" par la Société CIBA GEIGY, ou encore le poly(chlorure de méthacrylamidopropyltriméthylammonium) vendu sous la dénomination "POLYMAPTAC" par la Société TEXACO CHEMICALS.
(9) Les polymères quaternaires de vinylpyrrolidone et de vinylimidazole tel que par exemple les produits commercialisés sous les dénominations "LUVIQUAT FC 905, FC 550 et FC 370" par la Société B.A.S.F.
(10) Les polyamines comme le "POLYQUART H" vendu par la Société HENKEL, référencé sous le nom de "Polyéthylène Glycol (15) Tallow Polyamine" dans le dictionnaire CTFA.
(11) Le polymères de polyammonium quaternaire contenant des motifs récurrents répondant à la formule : 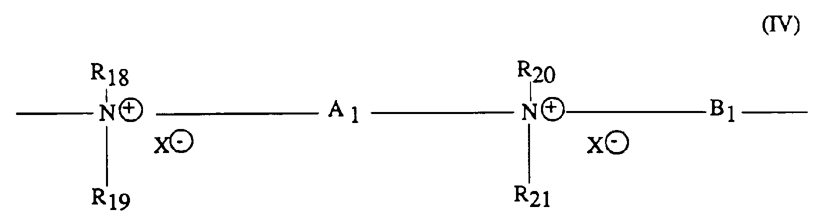 dans laquelle
   R₁₈, R₁₉. R₂₀ et R₂₁ étant identiques ou différents, représentent des radicaux aliphatiques, alicycliques ou arylaliphatiques contenant de 1 à 20 atomes de carbone ou des radicaux hydroxy alkylaliphatiques inférieurs, ou bien R₁₈, R₁₉, R₂₀ et R₂₁ ensemble ou séparément, constituent avec les atomes d'azote auxquels ils sont rattachés, des hétérocycles contenant éventuellement un second hétéroatome autre que l'azote, ou bien R₁₈, R_{19,} R₂₀ et R₂₁ représentent un radical alkyle en C₁-C₆ linéaire ou ramifié substitué par un groupement nitrile, ester, acyle, amide ou 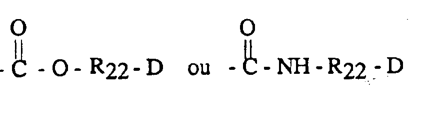 où
   R₂₂ est un alkylène et D un groupement ammonium quaternaire,
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
      désigne un anion dérivé d'un acide minéral ou organique.
   Xe et R₁₈ et R₂₀ peuvent former avec les deux atomes d'azote auxquels ils sont rattachés, un cycle pipérazi- nique; en outre, si A₁ désigne un radical alkylène ou hydroxyalkylène linéaire ou ramifié, saturé ou insaturé, B₁ peut également désigner un groupement : 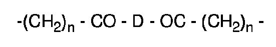 dans lequel D désigne :
   a) un reste de glycol de formule : -0 - Z - 0 - où Z désigne un radical hydrocarboné linéaire ou ramifié ou un groupement répondant aux formules : 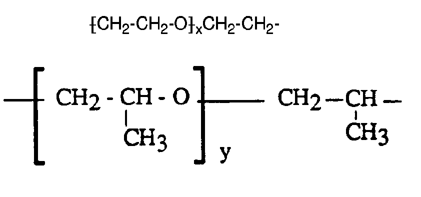 où
      x et y désignent un nombre entier de 1 à 4, représentant un degré de polymérisation défini et unique ou un nombre quelconque de 1 à 4 représentant un degré de polymérisation moyen :
   b) un reste de diamine bis-secondaire tel qu'un dérivé de pipérazine:
   c) un reste de diamine bis-primaire de formule : 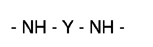 où
      Y désigne un radical hydrocarboné linéaire ou ramifié, ou bien le radical bivalent 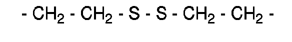
   d) un groupement uréylène de formule : 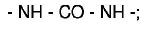 est un anion tel que chlorure ou bromure.

Ces Xe mères ont une masse moléculaire généralement comprise entre 1000 et 100 000.

Des polymères de ce type sont décrits en particulier dans les brevets français 2 320 330, 2 270 846,2 316 271, 2 336 434, et 2 413 907 et les brevets US-A-2 273 780, 2 375 853, 2 388 614, 2 454 547, 3 206 462, 2 261 002, 2 271 378, 3 874 870, 4 001 432, 3 929 990, 3 966 904, 4 005 193, 4 025 617, 4 025 627, 4 025 653, 4 026 945 et 4 027 020. (12) Les polymères de polyammonium quaternaires constitués de motifs de formule : 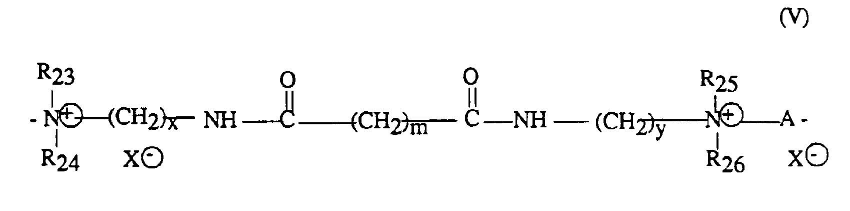 dans laquelle :
Rp₃, R₂₄, R₂₅ et R₂₆, identiques ou différents, représentent un atome d'hydrogène ou un radical méthyle, éthyle, propyle, β-hydroxyéthyle, p-hydroxypropyle ou -CH₂CH₂(OCH₂CH₂)poH, où
p est égal à 0 ou à un nombre entier compris entre 1 et 6, sous réserve que R₂₃, R₂₄, R₂₅ et R₂₆ ne représentent pas simultanément un atome d'hydrogène;
x et y, identiques ou différents, sont des nombres entiers compris entre 1 et 6;
m est égal à 0 ou à un nombre entier compris entre 1 et 34;
X désigne un atome d'halogène;
A désigne un radical d'un dihalogénure et représente de préférence

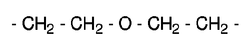

De tels composés sont décrits plus en détail dans la demande EP-A-122 324.

On peut par exemple citer parmi ceux-ci, les produits "MIRAPOL A 15", "MIRAPOL AD 1", "MIRAPOL AZ 1", "MIRAPOL 175", vendus par la Société MIRANOL.

D'autres polymères cationiques utilisables conformément à l'invention sont des polyalkylène imines en particulier des polyéthylèneimines, des polymères contenant des motifs vinylpyridine ou vinylpyridinium, des condensats de polyamines et d'épichlorhydrine, les polyuréylènes quaternaires et les dérivés de la chitine.

Les alkylgalactoside uronates de formule (I) sont présents dans les compositions conformes à l'invention, dans des proportions comprises entre 1 et 50 % en poids par rapport au poids total de la composition.

Les polymères cationiques définis précédemment sont présents dans les compositions conformes à l'invention dans des proportions comprises entre 0,01 et 10 % en poids par rapport au poids total de la composition.

Selon un mode de réalisation de l'invention la composition contient au moins un alkyl galactoside uronate de formule (I) telle que définie ci-dessus et dans laquelle lorsque R₂ représente un métal alcalin, celui-ci est différent du sodium, et au moins un polymère cationique tel que défini ci-dessus.

Selon un autre mode de réalisation de l'invention la composition contient l'association d'au moins un alkyl galactoside uronate de sodium et d'au moins une protéine quaternisée tels que définis ci-dessus.

Si les compositions selon l'invention ne sont pas utilisées pour le lavage des matières kératiniques, la concentration totale en tensioactifs anioniques de formule (I) est comprise entre 1 et 10 % et plus particulièrement entre 1 et 5 % en poids par rapport au poids total de la composition. Ces compositions sont utilisées notamment comme compositions à rincer ou non, appliquées avant ou après un shampooing, une coloration, une décoloration, une permanente, un défrisage ou dans des compositions de décoloration, de coloration, de permanente ou de défrisage.

Lorsque les compositions selon l'invention sont des compositions lavantes, elles contiennent les agents tensio- actifs de formule (I) dans une concentration totale comprise entre 4 et 50 % en poids et de préférence entre 8 et 40 % en poids par rapport au poids total de la composition.

Les compositions peuvent contenir outre les agents tensio-actifs anioniques de formule (I) d'autres agents tensio- actifs de nature anionique, non-ionique, amphotère, zwitterionique ou cationique.

Parmi les agents tensio-actifs anioniques, on peut citer les sels alcalins, les sels d'ammonium, les sels d'amines, les sels d'aminoalcools, les sels de magnésium des composés suivants : les acides gras, les alkylsulfates, les alkyléthersulfates, les alkylamidoéthersulfates, les alkylarylpolyéthersulfates, les monoglycérides sulfates; les alkylsulfonates, les alkyléthersulfonates, les alkylamides sulfonates, les alkylarylsulfonates, les oléfines sulfonates, les paraffines sulfonates; les alkylsulfosuccinates, les alkyléthersulfosuccinates, les alkylamides sulfosuccinates; les alkylsulfosuccinamates, les alkylsulfoacétates, les alkylétherphosphates; les acylsarcosinates, les acylglutamates, les N-acyltaurates; les iséthionates.

Le radical alkyle ou acyle de ces différents composés est généralement constitué par une chaîne carbonée comportant de 10 à 20 atomes de carbone.

On peut également utiliser des agents tensio-actifs faiblement anioniques, tels que les acides alkylamide ou alkyléthers carboxyliques polyoxyalkylénés, tels que ceux comportant 2 à 50 groupements oxyde d'éthylène.

Les agents tensio-actifs non-ioniques sont plus particulièrement choisis parmi les alcools, les a-diols, les alkylphénols et les acides gras polyéthoxylés ou polypropoxylés à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut citer plus particulièrement les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amines grasses polyéthoxylées ayant de préférence 2 à 30 moles d'oxyde d'éthylène; les esters d'acides gras du sorbitan oxyéthylénés ayant de préférence 2 à 30 moles d'oxyde d'éthylène; les esters d'acides gras de sucre, les esters d'acides gras du polyéthylèneglycol, les esters d'acides gras de glycols; les oxydes d'amines tels que les oxydes d'alkyl(Cᵢₒ-C₁ₐ) amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂ₒ)bétaïnes, les sulfobétaïnes, les alkyl(C₈-C₂₀)amidoalkyl(C₁-C₆)bétaïnes ou les alkyl(C₈-C2o)amidoalkyl(C1-C6)sulfobétaïnes.

On peut également citer les alkylpeptides, les alkylimidazolium bétaïnes.

Parmi les dérivés d'amines, on peut citer les produits commercialisés sous la dénomination "MIRANOL", tels que ceux décrits dans les brevets US-A-2.528.378 et 2.781.354 ou classés dans le dictionnaire CTFA, 3ème édition, 1982, sous les dénominations d'Amphocarboxyglycinates ou d'Amphocarboxypropionates.

Les agents tensio-actifs cationiques sont choisis parmi les sels d'ammonium quaternaires tels que les halogénures d'alkyl(C₈-C₂₂)triméthyl ammonium, les halogénures de dialkyl (C₈-C₂₂) diméthyl ammonium, les halogénures d'alkyl(C₈-C₂₂) diméthyl hydroxyéthyl ammonium.

Les co-tensio-actifs additionnels, peuvent représenter jusqu'à 50 % du poids total des agents tensio-actifs présents dans la composition.

Le pH des compositions conformes à l'invention est généralement compris entre 2 et 10,5, plus particulièrement entre 3 et 8.

Dans la mesure où le milieu cosmétiquement acceptable de la composition selon l'invention est un milieu aqueux, il peut être constitué uniquement par de l'eau ou par un mélange d'eau et d'un solvant cosmétiquement acceptable, tel que des alcools inférieurs en C↑ -C4 comme l'éthanol, l'isopropanol, le n-butanol; les alkylèneglycols comme le propy- lèneglycol; les éthers de glycols.

Les compositions selon l'invention peuvent se présenter sous forme de liquide plus ou moins épaissi, de gel, d'émulsion (lait ou crème), de lotion hydroalcoolique, de dispersion, de pain solide ou de mousse aérosol.

Les compositions sont par exemple des lotions, des laits ou des crèmes émollients, des lotions, des laits ou des crèmes pour les soins des matières kératiniques, des crèmes ou des laits démaquillants, des bases de fonds de teint, des lotions, des laits ou des crèmes antisolaires, des lotions, des laits ou des crèmes de bronzage artificiel, des crèmes ou des mousses de rasage, des lotions après rasage, des masques pour le visage, des produits de maquillage pour les yeux, des vernis à ongles, des fards et fonds de teint pour le visage, des shampooings, des produits pour le bain ou la douche, des compositions à rincer ou non, à appliquer avant ou après un shampooing, une coloration, une décoloration, une permanente ou un défrisage, des compositions de coloration, de décoloration, de permanente ou de défrisage des cheveux.

Les compositions conformes à l'invention peuvent également contenir en plus divers additifs tels des agents épaississants tels que des acides polyacryliques, des dérivés de cellulose, des esters d'acides gras et de polyéthylèneglycol; des séquestrants; des renforçateurs de mousse; des conservateurs; des parfums; des électrolytes; des corps gras tels que des alcools gras, des céramides, des huiles ou cires minérales, végétales, animales ou synthétiques; des filtres UV; des agents anti-radicaux libres; des agents nacrants; des biocides; des antibactériens; des agents anti-pelliculaires; des agents anti-séborrhéïques; des anti-parasitaires; des repellents; des colorants; des pigments; des oxydants; des réducteurs; des hydratants; des polymères anioniques, non ioniques ou amphotères; des vitamines; des a-hydroxyacides.

Le traitement des matières kératiniques se fait par application sur ces matières d'une quantité cosmétiquement acceptable d'une composition telle que définie ci-dessus.

Le procédé de lavage et/ou de conditionnement des matières kératiniques et en particulier des cheveux ou de la peau conforme à l'invention consiste à appliquer sur ces matières au moins une composition telle que définie ci-dessus, cette application étant suivie éventuellement d'une étape de rinçage à l'eau.

Les compositions de lavage peuvent être utilisées comme shampooing mais également comme gel-douche pour le lavage des cheveux et de la peau, auquel cas ils sont appliqués sur la peau et les cheveux humides qui sont rincés après application.

Lorsque les compositions sont utilisées pour le conditionnement des cheveux, elles sont appliquées sur les cheveux humides, après quoi on peut soit les sécher soit après un temps de pose de 1 à 10 minutes, les rincer à l'eau. On constate que les cheveux humides se démêlent bien.

Les exemples qui suivent sont destinés à illustrer l'invention sans pour autant présenter un caractère limitatif.

### EXEMPLE 1

On a préparé une lotion après-shampooing (à rincer) de composition suivante :

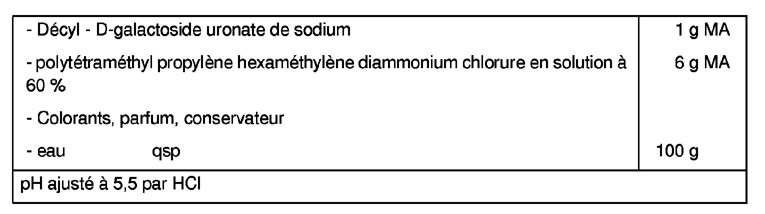

### EXEMPLE 2

On a préparé un bain moussant de composition suivante :

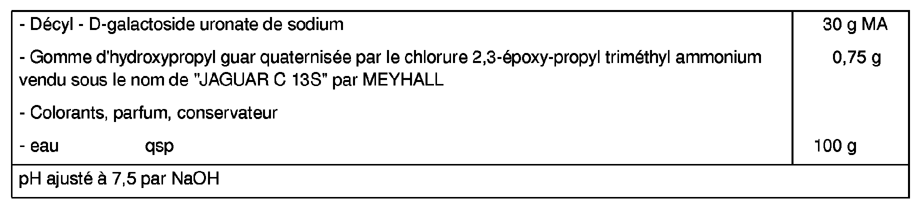

### EXEMPLE 3

On a préparé un shampooing de composition suivante :

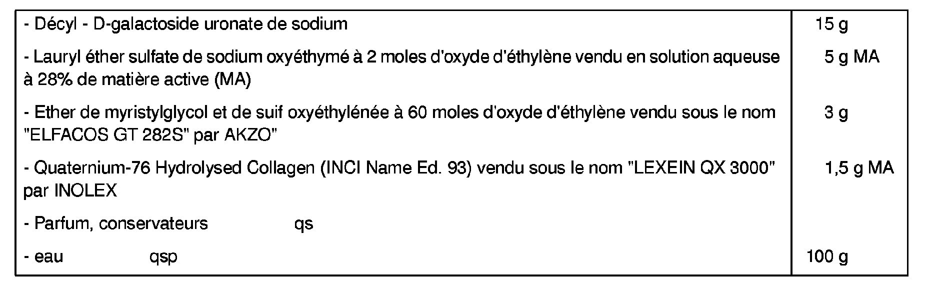

### EXEMPLE 4

On a préparé un shampooing de composition suivante :

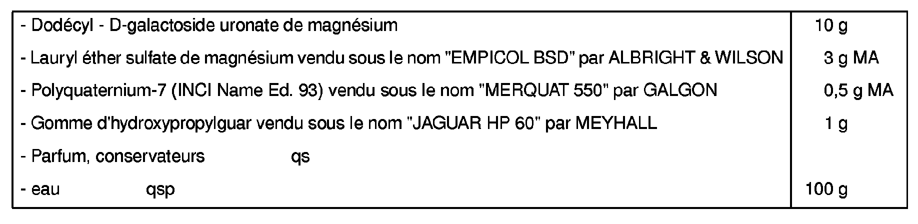

## Revendications

1. Composition cosmétique, caractérisée par le fait qu'elle contient dans un milieu aqueux cosmétiquement acceptable:
(A) au moins un agent tensio-actif anionique du type alkylgalactoside uronate de formule : 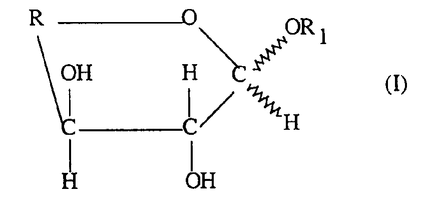
R₁ désigne un radical alkyle linéaire ou ramifié de 8 à 22 atomes de carbone.
R désigne un groupe 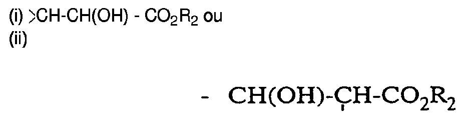 dont le carbone portant le groupe hydroxyle est relié à l'atome d'oxygène endocyclique; R₂ étant un métal alcalin à l'exception de Na, un métal alcalino-terreux ou un groupe ammonium quaternaire substitué ou non par des radicaux alkyle, hydroxyalkyle ou dérivé d'amino acide; et
(B) au moins un polymère cationique à l'exclusion des celluloses cationiques.

2. Composition selon la revendication 1, caractérisée par le fait que dans la formule (1), le radical R₂ désigne le potassium; le magnésium; le groupe ammonium quaternaire dérivé d'ammoniaque, de triéthanolamine, de monéthano- lamine, de 2-amino 2-méthyl 1,3-propane diol, de 2-amino 2-méthyl 1-propanol, d'histidine, d'arginine, ou de lysine.

3. Composition selon la revendication 1 ou 2, caractérisée par le fait que le composé de formule (I) est choisi parmi ceux pour lesquels R₁ désigne un alkyle en C₈-C₁₄.

4. Composition selon l'une quelconque des revendications 1 à 3, caractérisée par le fait que les composés de formule (I) sont choisis parmi ceux pour lesquels R₁ désigne un radical décyle.

5. Composition selon l'une quelconque des revendications 1 à 4, caractérisée en ce que le le polymère cationique est choisi parmi les polymères comportant des groupements amine primaire, secondaire, tertiaire et/ou quaternaire faisant partie de la chaîne polymère ou directement reliés à celle-ci et ayant un poids moléculaire compris entre 500 et environ 5 000 000, à l'exclusion des celluloses cationiques.

6. Composition selon la revendication 5, caractérisée en ce que les polymères comportant les groupements amine primaire, secondaire tertiaire et/ou quaternaire sont choisis parmi les protéines quaternisées et les polymères du type polyamine, polyaminoamide, ou du type polyammonium quaternaire dont le groupe ammonium fait partie d'un cycle ou de la chaîne polymérique ou est rattaché à la chaîne polymérique par un radical hydrocarboné.

7. Composition cosmétique, caractérisée par le fait qu'elle contient dans un milieu aqueux cosmétiquement acceptable:
(A) au moins un agent tensio-actif anionique du type alkylgalactoside uronate de formule : 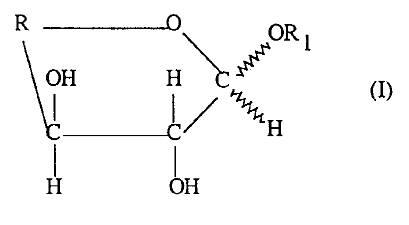
R₁ désigne un radical alkyle linéaire ou ramifié de 8 à 22 atomes de carbone.
R désigne un groupe
(i) CH-CH(OH) - CO₂R₂ ou
(ii) - CH(OH)-CH-C0₂R₂ dont le carbone portant le groupe hydroxyle est relié à l'atome d'oxygène endocyclique; R₂ étant l'hydrogène, un métal alcalin, un métal alcalino-terreux ou un groupe ammonium quaternaire substitué ou non par des radicaux alkyle, hydroxyalkyle ou dérivé d'amino acide; et
(B) au moins un polymère cationique choisi parmi les protéines quaternisées.

8. Composition selon la revendication 7, caractérisée par le fait que les composés de formule (I) sont choisis parmi ceux pour lesquels R₁ désigne un radical décyle.

9. Composition selon la revendication 6, 7 ou 8, caractérisée en ce que les protéines quaternisées sont choisies parmi les protéines suivantes :
- les hydrolysats de collagène portant des groupements triéthylammonium;
- les hydrolysats de collagène portant des groupements chlorure de triméthylammonium ou de diméthylstéa- rylammonium;
- les hydrolysats de protéines animales portant des groupements diméthylbenzylammonium;
- les hydrolysats de protéines portant sur la chaîne polypeptidique des groupements ammonium quaternaires comportant au moins un radical alkyle ayant de 1 à 18 atomes de carbone;
- les protéines végétales de soja quaternisées;
- les protéines quaternisées répondant à la formule : 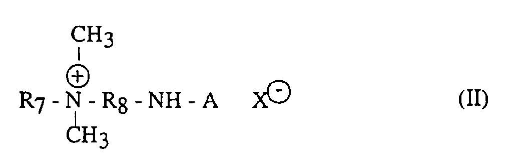 dans laquelle A désigne un reste de protéine dérivé d'hydrolysats de protéine de collagène, R₇ désigne un groupement lipophile comportant jusqu'à 30 atomes de carbone, R₈ représente un groupement alkylène ayant 1 à 6 atomes de carbone, XO représente un anion dérivé d'un acide organique ou minéral, et dont le poids moléculaire est compris entre 1500 et 10 000.

10. Composition selon la revendication 6, caractérisée en ce que les polymères du type polyamine, polyaminoamide ou polyammonium quaternaire sont choisis parmi
(1) les copolymères vinyl-pyrrolidonelacrylate ou méthacylate de dialkylaminoalkyle quaternisés ou non,
(2) les polysaccharides cationiques non cellulosiques,
(3) les polymères constitués de motifs pipérazinyle et de radicaux divalents alkylène ou hydroxyalkylène à chaînes droites ou ramifiées, éventuellement interrompues par des atomes d'oxygène, de soufre. d'azote ou par des cycles aromatiques ou hétérocyliques, ainsi que les produits d'oxydation et/ou de quaternisation de ces polymères,
(4) les polyaminoamides solubles dans l'eau préparés en particulier par polycondensation d'un composé acide avec une polyamine, éventuellement réticulés par une épihalohydrine, un diépoxyde, un dianhydride, un anhydride non saturé, un dérivé bis-insaturé, une bis-halohydrine, un bis-azétidinium, une bis-haloacyldiamine, un bis-halogénure d'alcoyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épihalohydrine, d'un diépoxyde ou d'un dérivé bis-insaturé; l'agent réticulant étant utilisé dans des proportions allant de 0,025 à 0,35 mole par groupement amine du polyaminoamide,
(5) les dérivés de polyaminoamides résultant de la condensation de polyalcoylène polyamines avec des acides polycarboxyliques suivie d'une alcoylation par des agents bifonctionnels,
(6) les polymères obtenus par réaction d'une polyalkylène polyamine comportant deux groupements amine primaire et au moins un groupement amine secondaire avec un acide dicarboxylique choisi parmi l'acide diglycolique, et des acides dicarboxyliques aliphatiques saturés ayant de 3 à 8 atomes de carbone, le rapport molaire entre la polyalkylène polyamine et l'acide dicarboxylique étant compris entre 0,8 : 1 et 1,4 : 1, le polyaminoamide en résulant étant amené à réagir avec l'épichlorhydrine dans un rapport molaire d'épichlorhydrine par rapport au groupement amine secondaire du polyaminoamide, compris entre 0,5 : 1 et 1,8 : 1,
(7) les cyclopolymères ayant un poids moléculaire de 20 000 à 3 000 000 tels que des homopolymères comportant comme constituant principal de la chaîne des motifs répondant aux formules (III) ou (III') 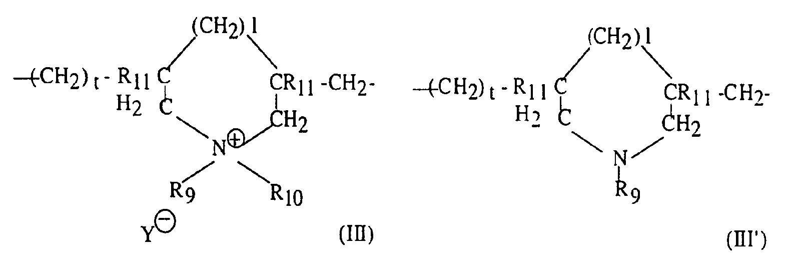 1 et t sont égaux à 0 ou 1, et la somme 1 + t = 1 , R₁₁ désigne hydrogène ou méthyle, Rg et R₁ désignent indépendamment l'un de l'autre un groupement alcoyle ayant de 1 à 22 atomes de carbone, un groupement hydroxyalcoyle dans lequel le groupement alcoyle a de préférence 1 à 5 atomes de carbone, un groupement amidoalcoyle inférieur, ou Rg et R₁₀ peuvent former conjointement avec l'atome d'azote auquel ils sont rattachés des groupements hétérocycliques tels que pipéridinyle ou morpholinyle, ainsi que les copolymères comportant des unités de formules (II ou (III') et des unités dérivées d'acrylamide ou de diacétone acrylamide, Y est un anion tel que bromure, chlorure, acétate, borate, citrate, tartrate, bisulfate, bisulfite, sulfate, phosphate,
(8) les homopolymères ou copolymères dérivés d'esters ou d'amides acrylique ou méthacrylique et comportant les motifs : 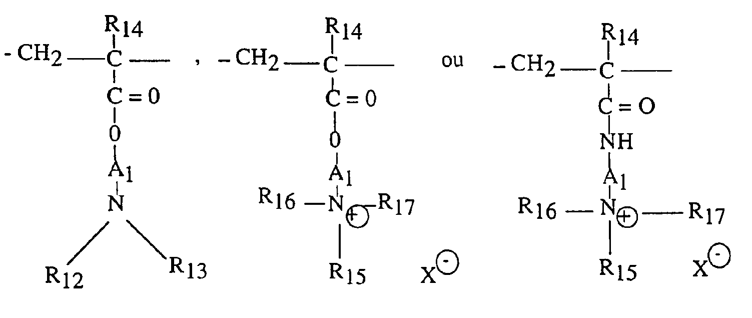 dans lesquels
R₁₄ désigne un atome d'hydrogène ou le radical méthyle,
A₁ est un groupe alkylène linéaire ou ramifié de 1 à 6 atomes de carbone ou un groupe hydroxyalkylène de 1 à 4 atomes de carbone,
R₁₅, R₁₆ et R₁₇, identiques ou différents, représentent un groupe alkyle ayant de 1 à 18 atomes de carbone ou un radical benzyle,
R₁₂ et R₁₃ représentent un atome d'hydrogène ou un groupe alkyle ayant de 1 à 6 atomes de carbone, X⊖ désigne un anion méthosulfate ou un halogénure tel que chlorure ou bromure,
(9) les polymères quaternaires de vinylpyrrolidone et de vinylimidazole,
(10) les polyamines,
(11) les polymères de polyammonium quaternaire de masse moléculaire comprise entre 1000 et 100 000 contenant des motifs récurrents répondant à la formule : 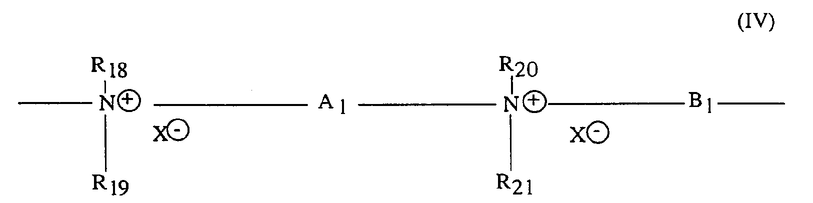 dans laquelle
R₁₈, R₁₉, R₂₀ et R₂₁ étant identiques ou différents, représentent des radicaux aliphatiques, alicycliques ou arylaliphatiques contenant de 1 à 20 atomes de carbone ou des radicaux hydroxy alkylaliphatiques inférieurs, ou bien R₁₈ et R₁₉ et R₂₀ et R₂₁ ensemble ou séparément, constituent avec les atomes d'azote auxquels ils sont rattachés, des hétérocycles contenant éventuellement un second hétéroatome autre que l'azote ou bien R₁₈, R₁₉, R₂₀ et R₂₁ représentent un radical alkyle en C₁-C₆ linéaire ou ramifié substitué par un groupement nitrile, ester, acyle, amide ou 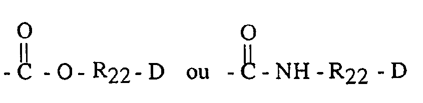 où
R₂₂ est un alkylène et D un groupement ammonium quaternaire,
A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⊖ désigne un anion dérivé d'un acide minéral ou organique,
A₁ et R₁₈ et R₁₉ peuvent former avec les deux atomes d'azote auxquels ils sont rattachés, un cycle pipé- razinique; en outre, si A₁ désigne un radical alkylène ou hydroxyalkylène linéaire ou ramifié, saturé ou insaturé, B₁ peut également désigner un groupement : 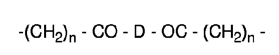 dans lequel D désigne :
a) un reste de glycol de formule : - O- Z - O- où Z désigne un radical hydrocarboné linéaire ou ramifié ou un groupement répondant aux formules : 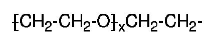 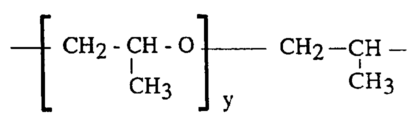 où
x et y désignent un nombre entier de 1 à 4, représentant un degré de polymérisation défini et unique ou un nombre quelconque de 1 à 4 représentant un degré de polymérisation moyen;
b) un reste de diamine bis-secondaire tel qu'un dérivé de pipérazine;
c) un reste de diamine bis-primaire de formule : 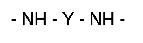 où
Y désigne un radical hydrocarboné linéaire ou ramifié, ou bien le radical bivalent 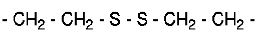
d) un groupement uréylène de formule : 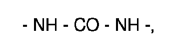
(12) les polymères de polyammonium quaternaires constitués de motifs de formule : 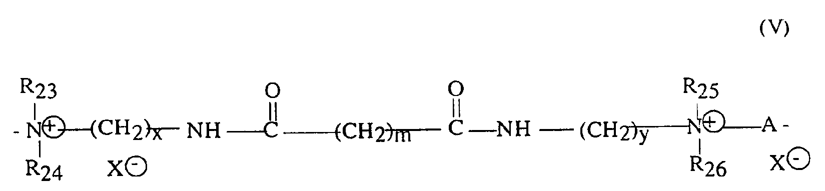 dans laquelle
: R₂₃, R₂₄, R₂₅ et R₂₆, identiques ou différents, représentent un atome d'hydrogène ou un radical méthyle éthyle, propyle, p-hydroxyéthy!e, p-hydroxypropyle ou -CH₂CH₂(OCH₂CH₂)pOH, où
p est égal à 0 ou à un nombre entier compris entre 1 et 6, sous réserve que R₂₃, R₂₄, R₂₅ et R₂₆ ne représentent pas simultanément un atome d'hydrogène;
x et y, identiques ou différents, sont des nombres entiers compris entre 1 et 6;
m est égal à 0 ou à un nombre entier compris entre 1 et 34;
X désigne un atome d'halogène;
A désigne un radical d'un dihalogénure et représente de préférence 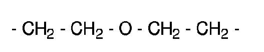
(13) les polyalkylèneimines; les polymères contenant des motifs vinylpyridine ou vinylpyridinium, les condensats de polyamines et d'épichlorhydrine, les polymères quaternaires, les dérivés de chitine.

11. Composition selon l'une quelconque des revendications 1 à 10, caractérisée par le fait que l'agent tensio-actif anionique de formule (I) est présent dans des proportions comprises entre 1 et 50 % en poids et le polymère cationique est présent dans des proportions comprises entre 0,01 et 10 % en poids ; les pourcentages en poids étant exprimés par rapport au poids total de la composition.

12. Composition de conditionnement des matières kératiniques selon l'une quelconque des revendications 1 à 11, caractérisée par le fait que la concentration en agents tensio-actifs anioniques de formule (I) est comprise entre 1 et 10 % en poids par rapport au poids total de la composition.

13. Composition de lavage des matières kératiniques selon l'une quelconque des revendications 1 à 11, caractérisée par le fait que la concentration en tensio-actifs anioniques de formule (I) est comprise entre 4 et 50 % en poids par rapport au poids total de la composition.

14. Composition selon l'une quelconque des revendications 1 à 13, caractérisée par le fait qu'elle contient en plus un co-tensio-actif additionnel, du type anionique, non-ionique, amphotère ou cationique dans une proportion allant jusqu'à 50 % du poids total en agents tensio-actifs.

15. Composition selon la revendication 14, caractérisée par le fait que le co-tensio-actif anionique additionnel est choisi parmi les acides gras, les alkylsulfates, alkyléthersulfates, alkylamidoéthersulfates, alkylarylpolyéthersulfates, monoglycérides sulfates; les alkylsulfonates, alkyléthersulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylétherphosphates; les acylsarcosinates, les acylglutamates, les N-acyltaurates; les iséthionates; le radical alkyle ou acyle étant constitué d'une chaîne carbonée comportant de 10 à 20 atomes de carbone ou bien des acides alkylamides ou alkyléthers carboxyliques polyoxyalkylénés.

16. Composition selon la revendication 14, caractérisée par le fait que le co-tensio-actif non-ionique additionnel est choisi parmi les alcools, les a-diols, les alkylphénols et les acides gras polyéthoxylés ou polypropoxylés à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30, les copolymères d'oxydes d'éthylène et de propylène; les condensats d'oxydes d'éthylène et de propylène sur des alcools gras; les amides gras polyoxyéthylénés; les amines grasses polyoxyéthylénés; les esters d'acides gras du sorbitan oxyéthylénés; les esters d'acides de sucre; les esters d'acides gras du polyéthylèneglycol; les esters d'acides gras des glycols; les oxydes d'amines.

17. Composition selon la revendication 14, caractérisée par le fait que le co-tensio-actif amphotère additionnel est choisi parmi. les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les a!kyi(C₈-C₂ₒ)bétaïnes, les sulfobétaïnes, les alkyl(Cs-C2o)amidoalkyl(C1-C6)bétaïnes ou les alkyl(C_{$}-C₂ₒ)amidoalkyl(C₁-C₆)sulfobétaïnes; les alkylpeptides; les alkylimidazolium bétaïnes.

18. Composition selon la revendication 14, caractérisée par le fait que le co-tensio-actif cationique est choisi parmi les sels d'ammonium quaternaire.

19. Composition selon l'une quelconque des revendications 1 à 18, caractérisée par le fait que le milieu cosmétiquement acceptable est constitué par de l'eau ou un mélange d'eau et d'un solvant cosmétiquement acceptable.

20. Composition selon l'une quelconque des revendications 1 à 19, caractérisée par le fait qu'elle se présente sous forme de liquide plus ou moins épaissi, de gel, d'émulsion, de lotion hydroalcoolique, de dispersion, de pain solide ou de mousse aérosol.

21. Composition selon l'une quelconque des revendications 1 à 20, caractérisée par le fait qu'elle contient en plus des additifs choisis parmi les renforçateurs de mousse, les épaississants, les séquestrants, les électrolytes, les parfums, les conservateurs, les alcools gras, les huiles ou cires minérales, végétales, animales ou synthétiques, les céramides, les filtres UV, les agents anti-radicaux libres, les agents nacrants, les biocides, les antibactériens, les agents anti-pelliculaires, les agents anti-séborrhéïques, les anti-parasitaires, les repellents, les colorants, les pigments, les oxydants, des réducteurs, les hydratants, les polymères anioniques, non ioniques ou amphotères, les vitamines, les a-hydroxyacides.

22. Utilisation de la composition telle que définie dans l'une quelconque des revendications 1 à 21 pour le traitement et/ou le lavage des matières kératiniques, particulièrement des cheveux et/ou de la peau.

23. Procédé de lavage et/ou de conditionnement cosmétique des cheveux ou de la peau, caractérisé par le fait qu'il consiste à appliquer sur la peau ou les cheveux une quantité efficace de composition selon l'une quelconque des revendications 1 à 21, cette application étant éventuellement suivie d'un rinçage à l'eau.

## Claims

1. Cosmetic composition, characterized in that it contains, in a cosmetically acceptable aqueous medium:
(A) at least one anionic surfactant of alkylgalactoside uronate type of formula: 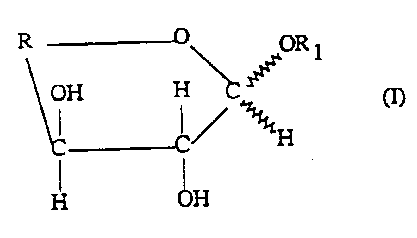 [lacuna]
R₁ denotes a linear or branched alkyl radical containing 8 to 22 carbon atoms,
R denotes a group 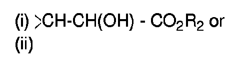 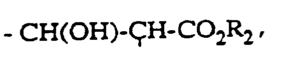 in which the carbon carrying the hydroxyl group is connected to the endocyclic oxygen atom; R₂ being an alkali metal with the exception of Na, an alkaline-earth metal or a quaternary ammonium group which is unsubstituted or substituted by alkyl or hydroxyalkyl radicals or derived from amino acid; and
(B) at least one cationic polymer, with the exception of cationic celluloses.

2. Composition according to Claim 1, characterized in that, in the formula (I), the radical R₂ denotes potassium; magnesium; or the quaternary ammonium group derived from ammonia, triethanolamine, monoethanolamine, 2-amino-2-methyl-1,3-propanediol, 2-amino-2-methyl-1-propanol, histidine, arginine or lysine.

3. Composition according to Claim 1 or 2, characterized in that the compound of formula (I) is chosen from those in which R₁ denotes a C₈-C₁₄ alkyl.

4. Composition according to any one of Claims 1 to 3, characterized in that the compounds of formula (I) are chosen from those in which R₁ denotes a decyl radical.

5. Composition according to any one of Claims 1 to 4, characterized in that the the [sic] cationic polymer is chosen from polymers containing primary, secondary, tertiary and/or quaternary amine groups which form part of the polymer chain or are directly connected to the latter and which have a molecular weight of between 500 and approximately 5,000,000, with the exception of cationic celluloses.

6. Composition according to Claim 5, characterized in that the polymers containing primary, secondary [lacuna] tertiary and/or quaternary amine groups are chosen from the quaternized proteins and the polymers of the polyamine or polyaminoamide type or of the quaternary polyammonium type in which the ammonium group forms part of a ring or of the polymer chain or is attached to the polymer chain by a hydrocarbon radical.

7. Cosmetic composition, characterized in that it contains, in a cosmetically acceptable aqueous medium:
(A) at least one anionic surfactant of alkylgalactoside uronate type of formula: 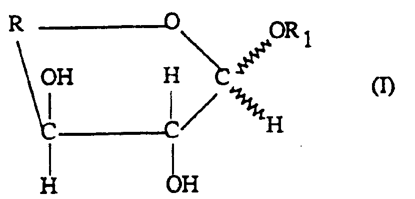 [lacuna]
R₁ denotes a linear or branched alkyl radical containing 8 to 22 carbon atoms, R denotes a group
(i) CH-CH(OH) - C0₂R₂ [sic] or
(ii) - CH(OH)-CH-C0₂R₂ [sic], in which the carbon carrying the hydroxyl group is connected to the endocyclic oxygen atom; R₂ being hydrogen, an alkali metal, an alkaline-earth metal or a quaternary ammonium group which is unsubstituted or substituted by alkyl or hydroxyalkyl radicals or derived from amino acid;
(B) at least one cationic polymer chosen from the quaternized proteins.

8. Composition according to Claim 7, characterized in that the compounds of formula (I) are chosen from those in which R₁ denotes a decyl radical.

9. Composition according to Claim 6, 7 or 8, characterized in that the quaternized proteins are chosen from the following proteins:
- collagen hydrolysates carrying triethylammonium groups;
- collagen hydrolysates carrying trimethylammonium or dimethylstearylammonium chloride groups;
animal protein hydrolysates carrying dimethylbenzylammonium groups;
- protein hydrolysates carrying, on the polypeptide chain, quaternary ammonium groups containing at least one alkyl radical having from 1 to 18 carbon atoms;
quaternized vegetable proteins from soya;
- quaternized proteins corresponding to the formula: 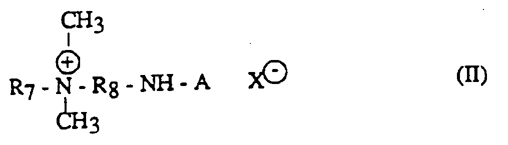 in which A denotes a protein residue derived from collagen protein hydrolysates, R₇ denotes a lipophilic group containing up to 30 carbon atoms, R₈ represents an alkylene group having 1 to 6 carbon atoms and X⁸ represents an anion derived from an organic or inorganic acid, and the molecular weight of which is between 1500 and 10,000.

10. Composition according to Claim 6, characterized in that the polymers of the polyamine, polyaminoamide or quaternary polyammonium type are chosen from
(1) optionally quaternized vinylpyrrolidone/dialkylaminoalkyl acrylate or methacrylate copolymers,
(2) noncellulose cationic polysaccharides,
(3) polymers consisting of piperazinyl units and of divalent, straight- or branched-chain alkylene or hydroxyalkylene radicals, optionally interrupted by oxygen, sulphur or nitrogen atoms or by aromatic or heterocyclic rings, as well as the oxidation and/or quaternization products of these polymers,
(4) water-soluble polyaminoamides prepared in particular by polycondensation of an acid compound with a polyamine, optionally crosslinked by an epihalohydrin, a diepoxide, a dianhydride, an unsaturated anhydride, a bisunsaturated derivative, a bishalohydrin, a bisazetidinium, a bishaloacyldiamine, an alkyl bishalide or alternatively by an oligomer resulting from the reaction of a bifunctional compound reactive with respect to a bishalohydrin, a bisazetidinium, a bishaloacyldiamine, an alkyl bishalide, an epihalohydrin, a diepoxide or a bisunsaturated derivative; the crosslinking agent being used in proportions ranging from 0.025 to 0.35 mol per amine group of the polyaminoamide,
(5) polyaminoamide derivatives resulting from the condensation of polyalkylenepolyamines with polycarboxylic acids followed by an alkylation by bifunctional agents,
(6) polymers obtained by reaction of a polyalkylene-polyamine containing two primary amine groups and at least one secondary amine group with a dicarboxylic acid chosen from diglycolic acid and saturated aliphatic dicarboxylic acids having from 3 to 8 carbon atoms, the molar ratio of polyalkylenepolyamine to dicarboxylic acid being between 0.8 : 1 and 1.4 : 1, the polyaminoamide resulting therefrom being brought to react with epichlorohydrin in a molar ratio of epichlorohydrin in relation to the secondary amine group of the polyaminoamide of between 0.5 : 1 and 1.8 : 1,
(7) cyclopolymers having a molecular weight of 20,000 to 3,000,000 such as the homopolymers containing, as principal constituent of the chain, units corresponding to the formulae (III) or (III'): 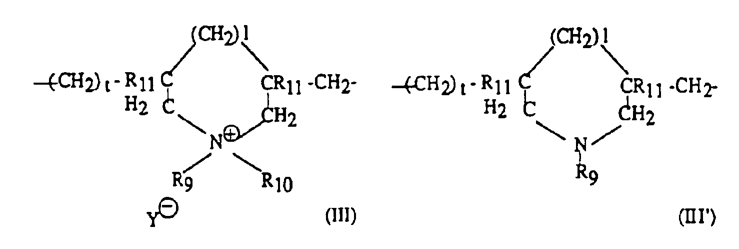 I and t are equal to 0 or 1, and the sum I + t = 1 , R₁₁ denotes hydrogen or methyl, R₉ and R₁₀ denote, independently of each other, an alkyl group having from 1 to 22 carbon atoms, a hydroxyalkyl group in which the alkyl group preferably has 1 to 5 carbon atoms or a lower amidoalkyl group, or R₉ and R₁₀ can form, jointly with the nitrogen atom to which they are connected, heterocyclic groups such as piperidinyl or morpholinyl, as well as copolymers containing units of formulae (III) or (III') and units derived from acrylamide or from diacetone acrylamide, Y [sic] is an anion such as bromide, chloride, acetate, borate, citrate, tartrate, bisulphate, bisulphite, sulphate or phosphate,
(8) homopolymers or copolymers derived from acrylic or methacrylic esters or amides and containing the units: 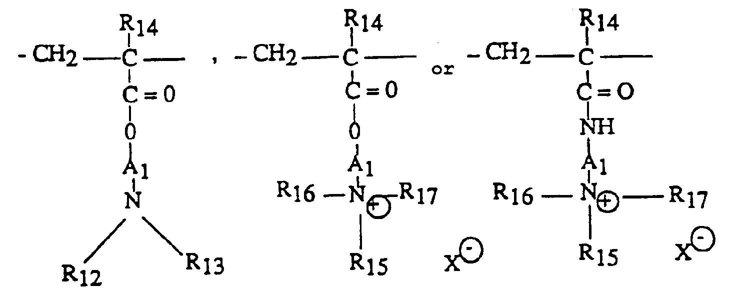 in which
R₁₄ denotes a hydrogen atom or the methyl radical, A₁ is a linear or branched alkylene group of 1 to 6 carbon atoms or a hydroxyalkylene group of 1 to 4 carbon atoms,
R_{15,} R₁₆ and R₁₇, which are indentical or different, represent an alkyl group having from 1 to 18 carbon atoms or a benzyl radical,
R₁₂ and R₁₃ represent a hydrogen atom or an alkyl group having from 1 to 6 carbon atoms,
X⁸ denotes a methosulphate anion or a halide such as chloride or bromide,
(9) quaternary polymers of vinylpyrrolidone and of vinylimidazole,
(10) polyamines,
(11) the quaternary polyammonium polymers with a molecular mass of between 1000 and 100,000 containing repeat units corresponding to the formula: 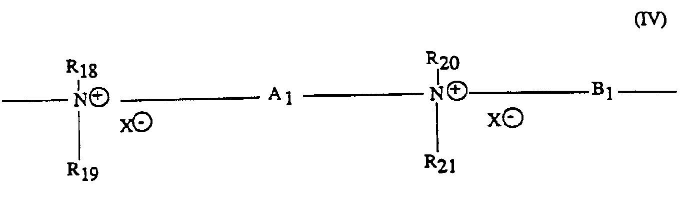 in which
R₁₈, R₁₉, R₂₀ and R₂₁, being identical or different, represent aliphatic, alicyclic or arylaliphatic radicals containing from 1 to 20 carbon atoms or lower hydroxy alkylaliphatic [sic] radicals or else R₁₈ and R₁₉ and R₂₀ and R₂₁, together or separately, form, with the nitrogen atoms to which they are connected, heterocycles which optionally contain a second heteroatom other than nitrogen or else R₁₈, R₁₉, R₂₀ and R₂₁ represent a linear or branched C₁-C₆ alkyl radical substituted with a nitrile, ester, acyl, amide or 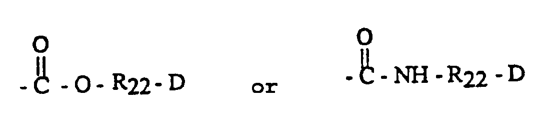 group, where
R₂₂ is an alkylene and D a quaternary ammonium group,
A₁ and B₁ represent polymethylene groups containing from 2 to 20 carbon atoms which may be linear or branched, saturated or unsaturated, and which may contain, connected to or inserted into the principal chain, one or more aromatic rings or one or more oxygen or sulphur atoms or sulphoxide, sulphone, disulphide, amino, alkylamino, hydroxyl, quaternary ammonium, ureido, amide or ester groups, and
X^{⊖} denotes an anion derived from an inorganic or organic acid,
A₁ and R₁₈ and R₁₉ may form, with the two nitrogen atoms to which they are connected, a piperazine ring;
additionally, if A₁ denotes a linear or branched, saturated or unsaturated alkylene or hydroxyalkylene radical, B₁ may also denote a group: 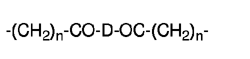 in which D denotes:
a) a glycol residue of formula: -O-Z-O-where Z denotes a linear or branched hydrocarbon radical or a group corresponding to the formulae: 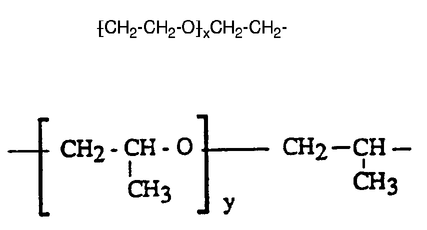 where x and y denote an integer from 1 to 4, representing a defined and single degree of polymerization or any number whatsoever from 1 to 4 representing an average degree of polymerization; b) a bis-secondary diamine residue such as a piperazine derivative;
c) a bis-primary diamine residue of formula: 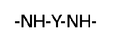 where Y denotes a linear or branched hydrocarbon radical, or else the bivalent radical 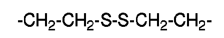
d) a ureylene group of formula: 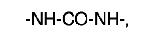
(12) quaternary polyammonium polymers consisting of units of formula: 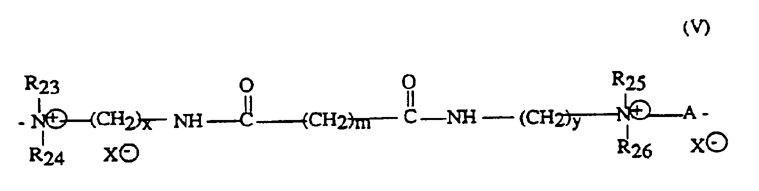 in which
R₂₃, R₂₄, R₂₅ and R₂₆, which are identical or different, represent a hydrogen atom or a methyl, ethyl, propyl, P-hydroxyethyl, P-hydroxypropyl or -CH₂CH₂(OCH₂CH₂)pOH radical, where
p is equal to 0 or an integer of between 1 and 6, with the proviso that R₂₃, R₂₄, R₂₅ and R₂₆ do not simultaneously represent a hydrogen atom;
x and y, which are identical or different, are integers of between 1 and 6;
m is equal to 0 or to an integer of between 1 and 34;
X denotes a halogen atom;
A denotes a dihalide radical and preferably represents 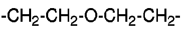
(13) polyalkyleneimines; polymers containing vinylpyridine or vinylpyridinium units, condensates of polyamines and of epichlorohydrin, quaternary polymers or chitin derivatives.

11. Composition according to any one of Claims 1 to 10, characterized in that the anionic surfactant of formula (I) is present in proportions of between 1 and 50% by weight and the cationic polymer is present in proportions of between 0.01 and 10% by weight; the percentages by weight being expressed with respect to the total weight of the composition.

12. Composition for conditioning keratinous substances according to any one of Claims 1 to 11, characterized in that the concentration of anionic surfactants of formula (I) is between 1 and 10% by weight with respect to the total weight of the composition.

13. Composition for washing keratinous substances according to any one of Claims 1 to 11, characterized in that the concentration of anionic surfactants of formula (I) is between 4 and 50% by weight with respect to the total weight of the composition.

14. Composition according to any one of Claims 1 to 13, characterized in that it furthermore contains an additional cosurfactant of anionic, nonionic, amphoteric or cationic type in a proportion ranging up to 50% of the total weight of surfactants.

15. Composition according to Claim 14, characterized in that the additional anionic cosurfactant is chosen from [lacuna] the fatty acids, alkyl sulphates, alkyl ether sulphates, alkylamidoether sulphates, alkylarylpolyether sulphates or monoglyceride sulphates; the alkylsulphonates, alkylethersulphonates, alkylamidesulphonates, alkylar- ylsulphonates, olefinsulphonates or paraffinsulphonates; the alkylsulphosuccinates, the alkylethersulphosuccinates or the alkylamidesulphosuccinates; the alkylsulphosuccinamates; the alkylsulphoace- tates; the alkyl ether phosphates; the acylsarcosinates, acylglutamates or N-acyltaurates; or the isethionates; the alkyl or acyl radical consisting of a carbon chain containing from 10 to 20 carbon atoms or else polyoxyalkylenated alkyl amide or alkyl ether carboxylic acids.

16. Composition according to Claim 14, characterized in that the additional nonionic cosurfactant is chosen from the polyethoxylated or polypropoxylated alcohols, a-diols, alkylphenols and fatty acids, with a fatty chain containing 8 to 18 carbon atoms, the number of ethylene oxide or propylene oxide groups being between 2 and 50 and the number of glycerol groups being between 2 and 30 [sic], the copolymers of ethylene oxide and of propylene oxide; the condensates of ethylene oxide and of propylene oxide with fatty alcohols; the polyoxyethylenated fatty amides; the polyoxyethylenated fatty amines; the oxyethylenated fatty acid esters of sorbitan; the fatty acid esters of sugar [sic]; the fatty acid esters of polyethylene glycols; the fatty acid esters of glycol; or the amine oxides.

17. Composition according to Claim 14, characterized in that the additional amphoteric cosurfactant is chosen from the derivatives of secondary or tertiary aliphatic amines, in which the aliphatic radical is a linear or branched chain containing 8 to 18 carbon atoms and which contains at least one water-solubilizing carboxylate, sulphonate, sulphate, phosphate or phosphonate anionic group; the (C₈-C₂₀)alkylbetaines, the sulphobetaines, the (C₈-C₂₀)alkylamido(Ci-C6)alkylbetaines or the (Cₛ-C₂ₒ)-alkylamido(C₁-C₆)alkylsulphobetaines; the alkylpeptides; or the alkylimidazolium betaines.

18. Composition according to Claim 14, characterized in that the cationic cosurfactant is chosen from the quaternary ammonium salts.

19. Composition according to any one of Claims 1 to 18, characterized in that the cosmetically acceptable medium consists of water or a mixture of water and of a cosmetically acceptable solvent.

20. Composition according to any one of Claims 1 to 19, characterized in that it is provided in the form of a more or less thickened liquid, a gel, an emulsion, an aqueouslalcoholic lotion, a dispersion, a solid bar or an aerosol foam.

21. Composition according to any one of Claims 1 to 20, characterized in that it furthermore contains additives chosen from foam reinforcers, thickeners, sequestering agents, electrolytes, fragrances, preservatives, fatty alcohols, mineral, vegetable, animal or synthetic oils or waxes, ceramides, UV screening agents, agents for combating free radicals, pearlescence agents, biocides, antibacterials, antidandruff agents, antiseborrheic agents, antiparasitic agents, repellents, dyes, pigments, oxidizing agents, reducing agents, moisturizers, anionic, nonionic or amphoteric polymers, vitamins or a-hydroxy acids.

22. Use of the composition as defined in any one of Claims 1 to 21 for treating and/or washing keratinous substances, particularly the hair and/or the skin.

23. Process for cosmetic washing and/or conditioning of the hair or of the skin, characterized in that it consists in applying an effective amount of composition according to any one of Claims 1 to 21 to the skin or the hair, this application optionally being followed by a rinsing with water.

## Patentansprüche

1. Kosmetische Zusammensetzung,
dadurch gekennzeichnet, daß
sie in einem wässrigen kosmetisch geeigneten Milieu enthält:
(A) mindestens ein anionisches oberflächenaktives Mittel vom Alkylgalactosiduronat-Typ der Formel: 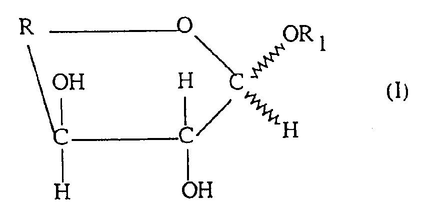 worin gilt:
R₁ bedeutet einen linearen oder verzweigten Alkylrest mit 8 bis 22 Kohlenstoffatomen;
R bedeutet eine Gruppe:
(i) ;CH-CH(OH) - C0₂R₂ oder
(ii) 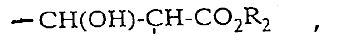 deren die Hydroxylgruppe aufweisender Kohlenstoff an das endozyklische Sauerstoffatom gebunden ist, worin R₂ ein Alkalimetall unter Ausschluß von Na, ein Erdalkalimetall oder eine quaternäre Ammoniumgruppe ist, die gegebenenfalls mit Alkyl- oder Hydroxyalkylresten substituiert oder von einer Aminosäure abgeleitet ist; sowie
(B) mindestens ein kationisches Polymer, ausgenommen kationische Cellulosen.

2. Zusammensetzung gemäß Anspruch 1,
dadurch gekennzeichnet, daß
in der Formel (I) der Rest R₂ Kalium, Magnesium oder eine quaternäre Ammoniumgruppe bedeutet, die von Ammoniak, Triethanolamin, Monoethanolamin, 2-Amino-2-methylpropan-1,3-diol, 2-Amino-2-methylpropan-1-ol, Histidin, Arginin oder Lysin abgeleitet ist.

3. Zusammensetzung gemäß Anspruch 1 oder 2,
dadurch gekennzeichnet, daß
die Verbindung der Formel (I) aus denjenigen ausgewählt ist, für welche R₁ einen C₈₋₁₄-Alkylrest bedeutet.

4. Zusammensetzung gemäß jedem der Ansprüche 1 bis 3,
dadurch gekennzeichnet, daß
die Verbindungen der Formel (I) aus denjenigen ausgewählt sind, für welche R₁ einen Decylrest bedeutet.

5. Zusammensetzung gemäß jedem der Ansprüche 1 bis 4,
dadurch gekennzeichnet, daß
das kationische Polymer aus Polymeren mit primären, sekundären, tertiären und/oder quaternären Amingruppen ausgewählt ist, die einen Teil der Polymerkette bilden oder direkt an diese gebunden sind und ein Molekulargewicht von 500 bis ca. 5 000 000 aufweisen, ausgenommen kationische Cellulosen.

6. Zusammensetzung gemäß Anspruch 5,
dadurch gekennzeichnet, daß
die Polymeren mit primären, sekundären, tertiären und/oder quaternären Amingruppen aus quaternierten Proteinen und aus Polymeren vom quaternären Polyamin-, Polyaminoamid- oder vom Polyammonium-Typ ausgewählt sind, deren quaternäre Ammoniumgruppe Teil eines Rings oder der Polymerkette darstellt oder an die Polymerkette über einen Kohlenwasserstoffrest gebunden ist.

7. Kosmetische Zusammensetzung,
dadurch gekennzeichnet, daß
sie in einem wässrigen kosmetisch geeigneten Milieu enthält:
(A) mindestens ein anionisches oberflächenaktives Mittel vom Alkylgalactosiduronat-Typ der Formel: 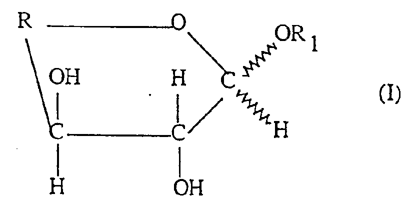 worin gilt:
R₁ bedeutet einen linearen oder verzweigten Alkylrest mit 8 bis 22 Kohlenstoffatomen;
R bedeutet eine Gruppe:
(i) CH-CH(OH) - C0₂R₂ oder
(ii) - CH(OH)-CH-C0₂R₂ , deren die Hydroxylgruppe aufweisender Kohlenstoff an das endozyklische Sauerstoffatom gebunden ist, worin R₂ ein Alkalimetall unter Ausschluß von Na, ein Erdalkalimetall oder eine quaternäre Ammoniumgruppe ist, die gegebenenfalls mit Alkyl- oder Hydroxyalkylresten substituiert oder von einer Aminosäure abgeleitet ist; sowie
(B) mindestens ein kationisches Polymer,das aus quaternierten Proteinen ausgewählt ist.

8. Zusammensetzung gemäß Anspruch 7,
dadurch gekennzeichnet, daß
die Verbindungen der Formel (I) aus denjenigen ausgewählt sind, für welche R₁ einen Decylrest bedeutet.

9. Zusammensetzung gemäß Anspruch 6, 7 oder 8,
dadurch gekennzeichnet, daß
die quaternierten Proteine aus den folgenden Proteinen ausgewählt sind:
- Kollagen-Hydrolysaten mit Triethylammonium-Gruppierungen;
- Kollagen-Hydrolysaten mit Trimethylammonium- oder Dimethylstearylammonium-Chlorid-Gruppierungen;
- Hydrolysaten tierischer Proteine mit Dimethylbenzylammonium-Gruppierungen;
- Hydrolysaten von Proteinen, die auf der Polypeptidkette quaternäre Ammoniumgruppen mit mindestens einem Alkylrest mit 1 bis 18 Kohlenstoffatomen aufweisen;
- quaternierten pflanzlichen Proteinen von Soja;
- quaternierten Proteinen der Formel: 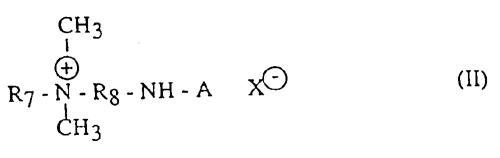 worin A einen Proteinrest, der aus Hydrolysaten von Kollagen-Protein abgeleitet ist, R₇ eine lipophile Gruppe mit bis zu 30 Kohlenstoffatomen, R₈ eine Alkylengruppe mit 1 bis 6 Kohlenstoffatomen und X- ein von einer organischen oder Mineralsäure abgeleitetes Anion darstellen, wobei deren Molekulargewicht 1500 bis 10 000 beträgt.

10. Zusammensetzung gemäß Anspruch 6,
dadurch gekennzeichnet, daß
die Polymeren vom quaternierten Polyamin-, Polyaminoamid- oder vom Polyammoniumtyp ausgewählt sind aus:
(1) Vinylpyrrolidon/Dialkylaminoalkylakrylat oder - methacrylat-Copolymeren, quaterniert oder nicht,
(2) kationischen nicht-cellulosischen Polysacchariden,
(3) Polymeren aus Piperazinyl-Einheiten und zweiwertigen Alkylen- oder Hydroxyalkylenresten mit geraden oder verzweigten Ketten, die gegebenenfalls mit Sauerstoff-, Schwefel- oder Stickstoffatomen oder durch aromatische oder heterozyklische Ringe unterbrochen sind, sowie den Oxidations- und/oder Quaternierungsprodukten dieser Polymeren,
(4) wasserlöslichen Polyaminoamiden, hergestellt insbesondere durch Polykondensation einer Säureverbindung mit einem Polyamin, welche gegebenenfalls mit einem Epihalohydrin, Diepoxid, Dianhydrid, ungesättigten Anhydrid, 2-fach ungesättigten Derivat, Bishalohydrin, einer Bisazetidiumverbindung, einem Bishaloacyldiamin, Alkylbishalogenid oder auch mit einem Oligomer aus der Reaktion einer bifunktionellen Verbindung, die gegenüber einem Bishalohydrin, einer Bisazetidiumverbindung, einem Bishaloacyldiamin, Alkylbishalogenid, Epihalohydrin, Diepoxid oder einem 2-fach ungesättigten Derivat reaktiv ist, vernetzt sind, wobei das Vernetzungsmittel in Mengenanteilen von 0,025 bis 0,35 Mol pro Amingruppe des Polyaminoamids verwendet wird,
(5) Derivaten von Polyaminoamiden aus der Kondensation von Polyalkylenpolyaminen mit Polycarboxylsäuren und anschließender Alkylierung mit bifunktionellen Agentien,
(6) Polymeren, erhalten aus der Reaktion eines Polyalkylenpolyamins, das zwei primäre Amingruppen und mindestens eine sekundäre Amingruppe aufweist, mit einer Dicarboxylsäure, ausgewählt aus Diglycolsäure und aus aliphatischen geästtigten Dicarboxylsäuren mit 3 bis 8 Kohlenstoffatomen, wobei das Molverhältnis zwischen dem Polyalkylenpolyamin und der Dicarboxylsäure 0,8:1 bis 1,4 : 1 beträgt, und wobei das daraus entstandene Polyaminoamid mit Epichlorhydrin in einem Molverhältnis von Epichlorhydrin, bezgoen auf die sekundäre Amingruppe des Polyaminoamids, von 0,5:1 bis 1,8:1 weiterreagiert wird,
(7) Cyclopolymeren mit einem Molekulargewicht von 20 000 bis 3 000 000 wie Homopolymere, die als hauptsächlichen Bestandteil der Kette Einheiten der Formel (111) oder (III') aufweisen: 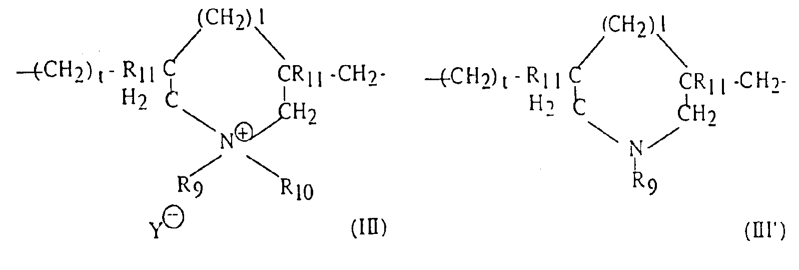 worin gilt:
I und t sind gleich 0 oder 1, und die Summe I + t = 1 , R₁₁ bedeutet Wasserstoff oder einen Methylrest, Rg und R₁₀ bedeuten, unabhängig voneinander, eine Alkylgruppe mit 1 bis 22 Kohlenstoffatomen, eine Hydroxialkylgruppe, worin die Alkylgruppe vorzugsweise 1 bis 5 Kohlenstoffatome aufweist, eine Amidoniedrigalkylgruppe, oder Rg und R₁₀ können zusammen mit dem Stickstoffatom, an das sie gebunden sind, heterozyklische Gruppierungen wie Piperidinyl- oder Morpholidinyl-Ringe bilden, wobei auch Copolymere zu nennen sind, die Einheiten der Formel (111) oder (III') und Einheiten aus Acrylamid oder Diacetonacrylamid aufweisen, Y ist ein Anion wie Bromid, Chlorid, Acetat, Borat, Zitrat, Tartrat, Bisulfat, Bisulfit, Sulfat und Phosphat,
(8) Homopolymere oder Copolymere, abgeleitet aus Estern oder Amiden der Acryl- oder Methacrylsäure, mit den Einheiten: 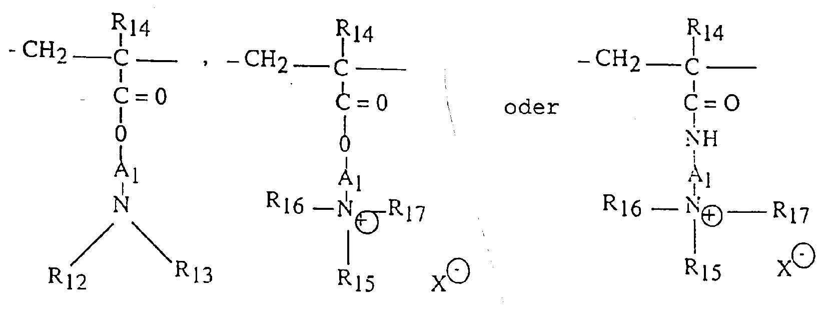 in denen
R₁₄ ein Wasserstoffatom oder einen Methylrest bedeutet, A₁ eine lineare oder verzweigte Alkylengruppe mit 1 bis 6 Kohlenstoffatomen oder eine Hydroxyalkylengruppe mit 1 bis 4 Kohlenstoffatomen ist, R₁₅, R₁₆ und R₁₇, gleich oder verschieden, eine Alkylgruppe mit 1 bis 18 Kohlenstoffatomen oder einen Benyzlrest darstellen,
R₁₂ und R₁₃ ein Wasserstoffatom oder eine Alkylgruppe mit 1 bis 6 Kohlenstoffatomen darstellen, und
X ein Methosulfat- oder Halogenidanion wie Chlorid oder Bromid bedeutet,
(9) quaternären Vinylpyrrolidon- und Vinylimidazol-Polymeren,
(10) Polyaminen,
(11) quaternären Polyammonium-Polymeren einer Molekularmasse von 1000 bis 100 000, enthaltend wiederkehrende Einheiten der Formel: 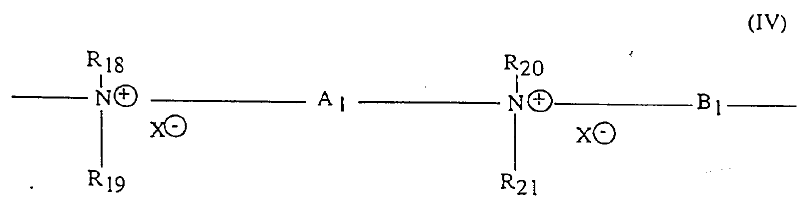 worin R₁₈, R₁₉, R₂₀ und R₂₁, gleich oder verschieden, aliphatische, alicyclische oder arylaliphatische Reste mit 1 bis 20 Kohlenstoffatomen oder aliphatische Hydroxyniedrigalkylreste darstellen, oder R₁₈, R₁₉, R₂₀ und R₂₁, jeweils zusammen oder getrennt, mit den Stickstoffatomen, an die sie gebunden sind, auch Heterozyklen bilden, die gegebenenfalls ein zweites, von Stickstoff verschiedenes Heteroatom enthalten, oder R₁₈, R₁₉, R₂₀ und R₂₁ auch einen linearen oder verzweigten C₁₋₆-Alkylrest darstellen, der mit einer Nitril-, Ester-, Acyl-, Amid- oder einer Gruppierung substituiert ist: 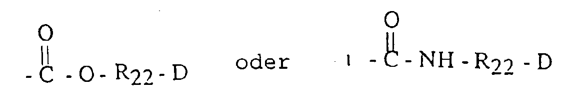 worin
R₂₂ eine Alkylengruppe und D eine quaternäre Ammoniumgruppe sind, und worin ferner gilt:
A₁ und B₁ stellen Polymethylengruppen mit 2 bis 20 Kohlenstoffatomen dar, die linear oder verzweigt, gesättigt oder ungesättigt sein und, gebunden an oder eingefügt in die Hauptkette, einen oder mehrere aromatische Ringe oder ein oder mehrere Sauerstoff-, Schwefelatome oder Sulfoxid-, Sulfon-, Disulfid-, Amino-, Alkylamino-, Hydroxyl-, quaternäre Ammonium-, Ureido-, Amid- oder Estergruppen enthalten können, und
X⁻ bedeutet ein von einer Mineral- oder organischen Säure abgeleitetes Anion,
A₁ und R₁₈ und R₁₉ können mit den beiden Stickstoffatomen, an die sie gebunden sind, auch einen Piperazin-Ring bilden; ausserdem kann Bᵢ, wenn A₁ einen linearen oder verzweigten, gesättigten oder ungesättigten Alkylen- oder Hydroxyalkylenrest bedeutet, auch eine Gruppierung bedeuten: 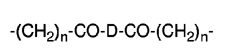 worin D bedeutet:
a) einen Glycolrest der Formel: -O-Z-O-,
worin Z einen linearen oder verzweigten Kohlenwasserstoffrest oder eine Gruppierung der Formeln bedeutet: 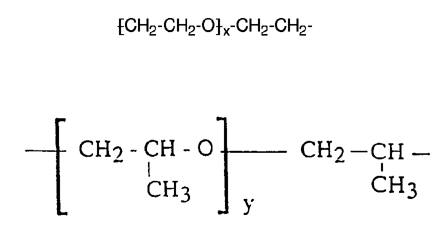 worin x und y eine ganze Zahl von 1 bis 4, die einen definierten und einzigen Polymerisationsgrad darstellt, oder irgendeine Zahl von 1 bis 4 bedeuten, die dann einen mittleren Polymerisationsgrad darstellt;
b) einen bissekundären Diaminrest wie ein Piperazinderivat,
c) einen bisprimären Diaminrest der Formel: 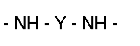 worin Y einen linearen oder verzweigten Kohlenwasserstoffrest oder auch den zweiwertigen Rest bedeutet: 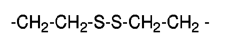
d) eine Harnstoff-Gruppierung der Formel: 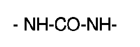
(12) quaternären Polyammonium-Polymeren aus Einheiten der Formel: 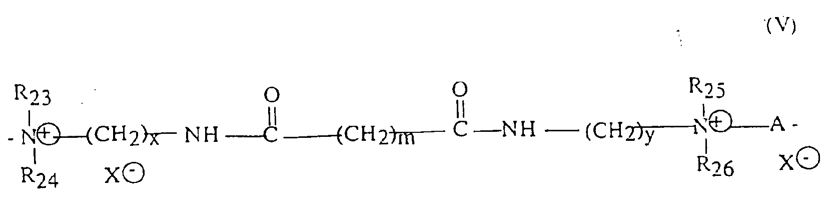 worin gilt: R₂₃, R₂₄, R₂₅ und R₂₆ stellen, gleich oder verschieden, ein Wasserstoffatom oder einen Methyl-, Ethyl-, Propyl-, β-Hydroxyethyl-, β-Hydroxypropyl- oder einen -CH₂CH₂(OCH₂CH₂)pOH-Rest dar, worin p gleich 0 oder eine ganze Zahl von 1 bis 6 ist, mit der Maßgabe, daß R₂₃, R₂₄,
R₂₅ und R₂₆ nicht gleichzeitig ein Wasserstoffatom darstellen;
x und y sind, gleich oder verschieden, ganze Zahlen von 1 bis 6;
m ist 0 oder eine ganze Zahl von 1 bis 34;
X bedeutet ein Halogenatom;
A bedeutet einen Rest eines Dihalogenids und stellt vorzugsweise dar: 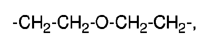
(13) Polyalkyleniminen, Polymeren, enthaltend Vinylpyridin- oder Vinylpyridinium-Einheiten, Kondensaten aus Polyaminen und Epichlorhydrin, quaternären Polymeren und aus Chitin-Derivaten.

11. Zusammensetzung gemäß einem der Ansprüche 1 bis 10, dadurch gekennzeichnet, daß das anionische oberflächenaktive Mittel der Formel (I) in Mengenanteilen von 1 bis 50 Gew.% und das kationische Polymer in Mengenanteilen von 0,01 bis 10 Gew.% vorhanden sind, wobei die Gewichtsprozentangaben auf das Gesamtgewicht der Zusammensetzung bezogen sind.

12. Zusammensetzung gemäß einem der Ansprüche 1 bis 11 zum Konditionieren keratinischer Materien, dadurch gekennzeichnet, daß
die Konzentration an anionischen oberflächenaktiven Mitteln der Formel (I) 1 bis 10 Gew.% beträgt, bezogen auf das Gesamtgewicht der Zusammensetzung.

13. Zusammensetzung gemäß einem der Ansprüche 1 bis 11 zum Waschen keratinischer Materien, dadurch gekennzeichnet, daß
die Konzentraton an anionischen oberflächenaktiven Mitteln der Formel (I) 4 bis 50 Gew.% beträgt, bezogen auf das Gesamtgewicht der Zusammensetzung.

14. Zusammensetzung gemäß einem der Ansprüche 1 bis 13,
dadurch gekennzeichnet, daß
sie ausserdem ein zusätzliches oberflächenaktives Co-Mittel vom anionischen, nicht-ionischen, amphoteren oder kationischen Typ in einem Mengenanteil bis insgesamt 50 Gew.% der oberflächenaktiven Mittel enthält.

15. Zusammensetzung gemäß Anspruch 14,
dadurch gekennzeichnet, daß
das zusätzliche oberflächenaktive Co-Mittel aus Fettsäuren, Alkylsulfaten, Alkylethersulfaten, Alkylamidoethersulfaten, Alkylarylpolyethersulfaten, Monoglyceridsulfaten, Alkylsulfonaten, Alkylethersulfonaten, Alkylamidsulfonaten, Alkylarylsulfonaten, Olefinsulfonaten, Paraffinsulfonaten, Alkylsulfosuccinaten, Alkylethersulfosuccinaten, Alkylamidsulfosuccinaten, Alkylsulfosuccinamaten, Alkylsulfoacetaten, Alkyletherphosphaten, Acylsarcosinaten, Acylglutamaten, N-Acyltauraten, Isethionaten, wobei der Alkyl- oder Acylrest aus einer Kohlenstoffkette mit 10 bis 20 Kohlenstoffatomen zusammengesetzt ist, oder auch aus polyoxalkylierten Alkylamid- oder Alkylethercarboxylsäuren ausgewählt ist.

16. Zusammensetzung gemäß Anspruch 14,
dadurch gekennzeichnet, daß
das nicht-ionische zusätzliche oberflächenaktive Co-Mittel aus polyethoxylierten oder polypropoxylierten, eine Fettkette mit 8 bis 18 Kohlenstoffatomen aufweisenden Alkoholen, a-Diolen, Alkylphenolen und Fettsäuren, wobei die Anzahl an Ethylenoxid- oder Propylenoxid-Gruppierungen 2 bis 50 und die Anzahl an Glyceryl-Gruppierungen 2 bis 30 betragen, aus Copolymeren aus Ethylen- und Propylenoxiden, aus Kondensaten von Ethylen- und Propylenoxiden an Fettalkohole, aus polyoxethylierten Fettamiden, polyoxethylierten Fettaminen, oxethylierten Sorbitanfettsäureestern, Zuckerfettsäureestern, Estern aus Fettsäuren und Glycolen sowie aus Aminoxiden ausgewählt ist.

17. Zusammensetzung gemäß Anspruch 14,
dadurch gekennzeichnet, daß
das zusätzliche amphotere oberflächenaktive Co-Mittel aus sekundären oder tertiären aliphatischen Aminderivaten, in denen der aliphatische Rest eine lineare oder verzweigte Kette mit 8 bis 18 Kohlenstoffatomen ist und mindestens eine anionische wasserlösliche Carboxylat-, Sulfonat-, Sulfat-, Phosphat- oder Phosphonatgruppe enthält, aus C_{$}_₂ₒ-Alkylbetainen, Sulfobetainen, Cₛ₋₂ₒ-Alkylamido-C₁₋₆-alkylbetainen oder aus C₈₋₂₀-Alkylamido-C₁₋₆- alkylsulfobetainen, Alkylpeptiden und aus Akylimidazoliumbetainen ausgewählt ist.

18. Zusammensetzung gemäß Anspruch 14,
dadurch gekennzeichnet, daß
das kationische oberfächenaktive Co-Mittel aus quaternären Ammoniumsalzen ausgewählt ist.

19. Zusammensetzung gemäß einem der Ansprüche 1 bis 18,
dadurch gekennzeichnet, daß
das kosmetisch geeignete Milieu aus Wasser oder aus einer Mischung aus Wasser und einem kosmetisch geeigneten Lösungsmittel zusammengesetzt ist.

20. Zusammensetzung gemäß einem der Ansprüche 1 bis 19,
dadurch gekennzeichnet, daß
sie in Form einer mehr oder weniger verdickten Flüssigkeit, eines Gels, einer Emulsion, hydroalkoholischen Lotion, Dispersion, eines Stücks Seife oder eines Aerosol-Schaums vorliegt.

21. Zusammensetzung gemäß einem der Ansprüche 1 bis 20,
dadurch gekennzeichnet, daß
sie zusätzlich Additive enthält, ausgewählt aus Schaumverstärkungsmitteln, Verdickungsmitteln, Sequestriermitteln, Elektrolyten, Parfüm-Produkten, Konservierungsstoffen, Fettalkoholen, mineralischen, pflanzlichen, tierischen oder synthetischen Ölen oder Wachsen, Ceramiden, UV-Filterstoffen, Abfangmitteln für freie Radikale, Bioziden, antibakteriellen Mitteln, Antischuppenmitteln, antiseborrheischen Mitteln, antiparasitären Mitteln, Abschreckmitteln, Farbstoffen, Pigmenten, Oxidations-, Reduktionsmitteln, Hydratisiermitteln, anionischen, nicht-ionischen oder amphoteren Polymeren, Vitaminen und aus a-Hydroxisäuren.

22. Verwendung der in jedem der Ansprüche 1 bis 21 definierten Zusammensetzung zur Behandlung und/oder zum Waschen keratinischer Materien, insbesondere der Haare und/oder der Haut.

23. Kosmetisches Verfahren zum Waschen und/oder Konditionieren der Haare oder der Haut, dadurch gekennzeichnet, daß
man auf die Haut oder die Haare eine wirksame Menge einer Zusammensetzung gemäß jedem der Ansprüche 1 bis 21 aufbringt und nach dieser Aufbringung gegebenenfalls eine Spülung mit Wasser folgen läßt.
